# EUROPÄISCHE PATENTANMELDUNG

(11) **EP 4 599 925 A1**
(43) Veröffentlichungstag der Anmeldung: **13.08.2025**
(21) Anmeldenummer: 25156348.2
(22) Anmeldetag: 06.02.2025
(51) Int. Cl.: B01D 61/12, A61M 1/16, C02F 1/44

(54) **PERMEATVERSORGUNGSSYSTEM UND ZUGEHÖRIGES BETRIEBSVERFAHREN**

(30) Priorität: 07.02.2024 DE 102024103413
(71) Anmelder: B. Braun Avitum AG, 34212 Melsungen (DE)
(72) Erfinder: LIEB, Nino, 34212 Melsungen (DE); ODERNHEIMER, Philipp, 34212 Melsungen (DE)
(74) Vertreter: Tergau & Walkenhorst Intellectual Property GmbH

(57) **Zusammenfassung**

Ein für hohe Versorgungsicherheit und Zuverlässigkeit bei zugleich hoher Energieeffizienz ausgelegtes Permeatversorgungssystem (2) für medizinische Anwendungen, umfasst:
• eine Wasseraufbereitungsanlage (90) mit mindestens einer integrierten Pumpe (5) zur Produktion von Permeat,
• ein eingangsseitig über einen Permeatteiler (16) an die Wasseraufbereitungsanlage (90) angeschlossenes Ringleitungssystem mit mindestens zwei parallel geschalteten Ringleitungen (10, 12), die ausgangsseitig über eine Rücklaufvorrichtung (50) an die Wasseraufbereitungsanlage (90) angeschlossen sind,
• eine Steuer- oder Regeleinheit (80),

wobei die Rücklaufvorrichtung (50) für jede der Ringleitungen (10, 12) folgende Komponenten umfasst:
• einen Ringleitungsrücklauf (101, 105), der sich in zwei parallel geschaltete Leitungsstränge verzweigt, wobei der eine Leitungsstrang einen Durchflussbegrenzer (111, 114) und der andere Leitungsstrang ein oberhalb eines eingestellten Öffnungsdrucks öffnendes Druckhalteventil (103, 107) aufweist,
• einen Drucksensor (110, 113), der stromaufwärts des Durchflussbegrenzers (111, 114) und des Druckhalteventils (103, 107) den Druck im Ringleitungsrücklauf (101, 105) misst,

und wobei in der Steuer- oder Regeleinheit (80) ein Steuer- oder Regelalgorithmus implementiert ist, der im Normalbetrieb anhand eines von einem der Drucksensoren (110, 113) gemessenen Drucks die Pumpe (5) ansteuert

## Beschreibung

Die vorliegende Erfindung betrifft das Gebiet der medizinischen Wasseraufbereitungssysteme. Insbesondere bezieht sie sich auf ein Permeatversorgungssystem, das für die Bereitstellung von hochwertigem Permeat in medizinischen Anwendungen, wie beispielsweise in Dialysegeräten, eingesetzt wird. Des Weiteren bezieht sich die Erfindung auf ein Verfahren zum Betreiben eines Permeatversorgungssystems.

Bisherige Permeatversorgungssysteme im medizinischen Umfeld verwenden Ringleitungssysteme, die an Wasseraufbereitungsanlagen, insbesondere an Umkehrosmoseanlagen, angeschlossen sind. Speziell bei der Verwendung eines "doppelten Ringleitungssatzes" mit zwei hydraulisch parallel geführten Ringleitungen bestehen Herausforderungen bezüglich der Rückführung und Regelung des Permeatflusses sowie der genauen Steuerung der Produktionspumpe, die den Druck und die Permeatproduktion beeinflusst. Bisherige Systeme lassen allenfalls einen ungeregelten oder volumenstromgeregelten Betrieb der Wasseraufbereitungsanlage zu.

Aufgabe der vorliegenden Erfindung ist es, ein Permeatversorgungssystem mit einem doppelten oder mehrfachen Ringleitungssatz bereitzustellen, das für einen zuverlässigen Betrieb mit hoher Versorgungssicherheit und betrieblicher Sicherheit bei dennoch hoher Energieeffizienz und mit möglichst einfachem Aufbau ausgelegt ist. Des Weiteren soll ein zugehöriges Betriebsverfahren angegeben werden.

Die genannte Aufgabe wird erfindungsgemäß gelöst durch ein Permeatversorgungssystem mit den Merkmalen des Anspruchs 1. Das zugehörige Betriebsverfahren ist in Anspruch 9 definiert.

Demnach ist ein Permeatversorgungssystem für medizinische Anwendungen vorgesehen, umfassend:
- eine Wasseraufbereitungsanlage mit mindestens einer integrierten Pumpe zur Produktion von Permeat,
- ein eingangsseitig über einen Permeatteiler an die Wasseraufbereitungsanlage angeschlossenes Ringleitungssystem mit mindestens zwei parallel geschalteten Ringleitungen, die ausgangsseitig über eine Rücklaufvorrichtung an die Wasseraufbereitungsanlage angeschlossen sind,
- eine Steuer- oder Regeleinheit,
wobei die Rücklaufvorrichtung für jede der Ringleitungen folgende Komponenten umfasst:
- einen Ringleitungsrücklauf, der sich in zwei parallel geschaltete Leitungsstränge verzweigt, wobei der eine Leitungsstrang einen Durchflussbegrenzer und der andere Leitungsstrang ein oberhalb eines eingestellten Öffnungsdrucks öffnendes Druckhalteventil aufweist,
- einen Drucksensor, der stromaufwärts des Durchflussbegrenzers und des Druckhalteventils den Druck im Ringleitungsrücklauf misst,
und wobei in der Steuer- oder Regeleinheit ein Steuer- oder Regelalgorithmus implementiert ist, der im Normalbetrieb anhand eines von einem der Drucksensoren gemessenen Drucks die Pumpe ansteuert.

Das System integriert eine Wasseraufbereitungsanlage mit einer oder mehreren Pumpen, um Permeat zu erzeugen. Über einen Permeatteiler ist ein Ringleitungssystem an die Wasseraufbereitungsanlage angeschlossen. Dieses Ringleitungssystem besteht aus mindestens zwei parallel geschalteten Ringleitungen, die über eine Rücklaufvorrichtung mit der Wasseraufbereitungsanlage verbunden sind.

Die Rücklaufvorrichtung jeder Ringleitung besteht aus einem Ringleitungsrücklauf, der sich in zwei parallel geschaltete Leitungsstränge verzweigt. Einer dieser Stränge beinhaltet einen Durchflussbegrenzer, während der andere Strang ein Druckhalteventil (Überströmventil) aufweist. Ein Drucksensor misst den Druck stromaufwärts des Durchflussbegrenzers und des Druckhalteventils im Ringleitungsrücklauf.

Die Steuer- oder Regeleinheit des Systems implementiert einen Steuer- oder Regelalgorithmus, der im Normalbetrieb anhand der Druckdaten, die von einem der Drucksensoren gemessen werden, die Pumpe steuert. Bevorzugt wird dabei die Drehzahl der Pumpe in Abhängigkeit vom Druck, der mittels einer der Drucksensoren gemessen wird, geregelt. Dies ermöglicht eine präzise Anpassung der Pumpenleistung entsprechend dem gemessenen Druck.

Diese Ausgestaltung ermöglicht unter anderem eine präzise Pumpensteuerung. Durch die Verwendung von Drucksensoren und eines spezifischen Algorithmus in der Steuereinheit kann die Pumpe genau an den gemessenen Druck angepasst werden. Das führt zu einer präzisen und effizienten Produktion von Permeat.

Des Weiteren ist eine optimierte Rückführung des Permeats ermöglicht: Die Struktur des Ringleitungsrücklaufs mit Durchflussbegrenzern und Druckhalteventilen gewährleistet eine effektive Rückführung des Permeats in die Wasseraufbereitungsanlage. Das minimiert Verluste und optimiert die Ressourcennutzung.

Vorteilhafterweise ist der Steuer- oder Regelalgorithmus so beschaffen, dass er in einer Initialisierungsphase genau einen der Drucksensoren für die Steuerung der Pumpe im anschließenden Normalbetrieb auswählt. Der ausgewählte Drucksensor ist bevorzugt derjenige, der in der Initialisierungsphase den niedrigsten Druck misst. Dadurch wird sichergestellt, dass der Druck in den anderen Ringleitungen eher höher ist, so dass in allen Ringleitungen ein hinreichender Mindestdurchsatz gewährleistet ist.

In bevorzugter Ausgestaltung münden die Ringleitungsrückläufe über einen Permeatsammler in einen gemeinsamen Rücklauf. Alternativ können getrennte Permeatrückläufe in einen gemeinsamen Tank vorgesehen sein.

Wenn in den Leitungsstrang mit dem Durchflussbegrenzer ein Rückschlagventil geschaltet ist, werden auch in ungünstigen Betriebszuständen Rückzirkulationen von Permeat im Ringleitungssystem vermieden.

In einer bevorzugten Ausgestaltung ist das Druckhalteventil ein Überströmventil und weist eine auf einen Ventilteller einwirkende Feder, insbesondere eine Druckfeder auf, die dem Mediumdruck entgegenwirkt, um unterhalb des Öffnungsdrucks das Überströmventil geschlossen zu halten.

In einer alternativen Ausgestaltung des Grundprinzips sind die Druckhalteventile hinsichtlich ihres Öffnungsdrucks elektrisch justierbar. Der Steuer- oder Regelalgorithmus wirkt dann bevorzugt derart auf die Druckhalteventile ein, dass der gleiche Druck in allen Ringleitungsrückläufen angestrebt wird. In diesem Fall regelt der Steuer- und Regelalgorithmus neben dieser Ventileinstellung auch weiterhin die Pumpendrehzahl, aber es ist nun egal, welcher der beiden bzw. mehreren Drucksensoren den Prozesswert liefert. Sollte die Druckangleichung nicht möglich sein, erfolgt wieder eine Druckregelung basierend auf dem niedrigsten in einer vorherigen Initialisierungsphase gemessenen Druck.

Die Verwendung einer Umkehrosmoseanlage als Wasseraufbereitungsanlage innerhalb des Permeatversorgungssystems bietet mehrere Vorteile: Umkehrosmoseanlagen sind bekannt für ihre hohe Filtrationsleistung. Sie entfernen effektiv Verunreinigungen, Schwermetalle, Salze und andere Partikel aus dem Wasser. Dadurch produzieren sie hochwertiges und sauberes Permeat, das den Anforderungen medizinischer Anwendungen entspricht. Die Umkehrosmose gewährleistet eine konstante Qualität des erzeugten Permeats. Das System bietet eine zuverlässige Performance. Die Effizienz der Umkehrosmoseanlage verringert den Bedarf an weiteren Reinigungsstufen oder zusätzlichen Behandlungen des Wassers. Das spart Zeit, Ressourcen und mögliche Komplikationen im System.

In Bezug auf das Verfahren wird die Eingangs genannte Aufgabe gelöst durch ein Verfahren zum Betreiben eines Permeatversorgungssystems für medizinische Anwendungen, umfassend
- eine Wasseraufbereitungsanlage mit mindestens einer integrierten Pumpe zur Produktion von Permeat,
- ein eingangsseitig über einen Permeatteiler an die Wasseraufbereitungsanlage angeschlossenes Ringleitungssystem mit mindestens zwei parallel geschalteten Ringleitungen, die ausgangsseitig über eine Rücklaufvorrichtung an die Wasseraufbereitungsanlage angeschlossen sind,
wobei die Rücklaufvorrichtung für jede der Ringleitungen folgende Komponenten umfasst:
- einen Ringleitungsrücklauf, der sich in zwei parallel geschaltete Leitungsstränge verzweigt, wobei der eine Leitungsstrang einen Durchflussbegrenzer und der andere Leitungsstrang ein oberhalb eines eingestellten Öffnungsdrucks öffnendes Druckhalteventil aufweist,
- einen Drucksensor, der stromaufwärts des Durchflussbegrenzers und des Druckhalteventils den Druck im Ringleitungsrücklauf misst,
und wobei im Normalbetrieb ein von einem der Drucksensoren gemessener Druck als Prozessvariable für die Ansteuerung der Pumpe verwendet wird.

Dabei ist es besonders vorteilhaft, wenn in einer Initialisierungsphase genau einer der Drucksensoren für die Steuerung der Pumpe im anschließenden Normalbetrieb ausgewählt wird. Vorteilhafterweise wird der derjenige Drucksensor ausgewählt, der in der Initialisierungsphase den niedrigsten Druck misst.

Die zur Vorrichtung genannten Merkmale, Varianten, Aufgaben und Vorteile übertragen sich analog auf das Verfahren und umgekehrt.

Mehrere Ausführungsbeispiele der Erfindung werden nachfolgend unter Bezugnahme auf die beigefügten Zeichnungen näher erläutert. Es zeigt:
- FIG. 1: einen schematischen Überblick über ein konventionelles Permeatversorgungssystem mit einem doppelten Ringleitungssatz,
- FIG. 2: eine Rücklaufvorrichtung für ein Permeatversorgungssystem gemäß FIG. 1,
- FIG. 3: ein erfindungsgemäß weiterentwickeltes Permeatversorgungssystem mit einem doppelten Ringleitungssatz,
- FIG. 4: eine weiterentwickelte Rücklaufvorrichtung für ein Permeatversorgungssystem gemäß FIG. 3,
- FIG. 5: einen Schnitt durch ein Druckhalteventil zum Einsatz in einer Rücklaufvorrichtung gemäß FIG. 4,
- FIG. 6: ein Flussdiagramm für ein Steuerungs- oder Regelungsverfahren für ein Permeatversorgungssystem gemäß FIG. 3, insbesondere mit einer Rücklaufvorrichtung gemäß FIG. 4,
- FIG. 7: eine Variation der aus FIG. 4 bekannten Rücklaufvorrichtung, und
- FIG. 8: eine weitere Variation der Rücklaufvorrichtung.

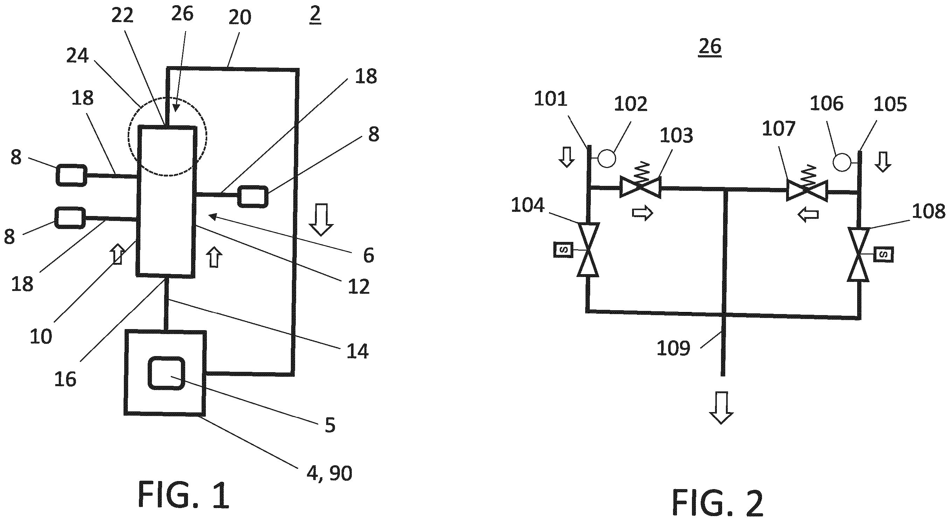

FIG. 1 zeigt rein schematisch ein Permeatversorgungssystem 2, insbesondere für medizinische Dialyseanwendungen, mit einem doppelten Ringleitungssatz. Eine Umkehrosmoseanlage 4 mit einer integrierten Pumpe 5 produziert hochreinreines Wasser, auch Permeat genannt, und stellt dieses über ein Ringleitungssystem 6 einer Anzahl von Verbrauchern 8 oder Abnehmern zur Verfügung. Nicht abgenommenes Permeat wird zum Zweck der erneuten Aufbereitung zur Umkehrosmoseanlage 4 zurückgeführt.

Hier im Beispiel sind zwei strömungstechnisch parallel geschaltete Ringleitungen 10, 12 vorgesehen, die beispielsweise durch verschiedene Räume eines Gebäudekomplexes (Krankenhaus, Dialysezentrum etc.) laufen. Dazu ist an den Vorlauf 14 der Anlage ein Permeatteiler 16, hier beispielsweise in Gestalt eines Y- oder T-Trennstücks oder Verteilers, angeschlossen, welcher den gesamten Permeatvolumenstrom in zwei Teilströme aufteilt. Die Teilströme fließen dann durch die erste Ringleitung 10 und die zweite Ringleitung 12 und werden dort über abzweigende Entnahmeleitungen 18 an die jeweiligen Verbraucher 8 oder Abnehmer verteilt. Die gegebenenfalls durch Entnahme reduzierten Teilströme werden durch die beiden Ringleitungen 10, 12 zurück in den Rücklauf 20 geleitet, welcher das nichtabgenommene Permeat sammelt und in die Umkehrosmoseanlage 4 zurückführt. Dabei erfolgt im dargestellten Beispiel eine Zusammenführung der beiden Ringleitungen 10, 12 in einen gemeinsamen Rücklauf 20 durch einen Permeatsammler 22, hier beispielsweise in Gestalt einer Y- oder T-Vereinigung.

Alternativ können die zwei Ringleitungen 10, 12 auch separat in einen als Zwischenspeicher wirksamen Tank geführt werden und müssen nicht zwangsläufig über einen Permeatsammler 22 zusammengeführt werden (oder man sieht den Tank als Sammler an). In anderen Ausführungsformen können der Permeatteiler 16 und/oder der Permeatsammler 22 Teil der Umkehrosmoseanlage 4 sein. Das dargestellte Prinzip der Teilung und Wiedervereinigung des Permeatvolumenstroms lässt sich in offensichtlicher Weise auf drei oder mehr parallele Ringleitungen verallgemeinern. Alle hier am Beispiel von zwei Ringleitungen 10, 12 gemachten Aussagen lassen sich daher im Sinne von mindestens zwei Ringleitungen verstehen.

Die in FIG. 1 nur schematisch dargestellte Rücklaufsektion 24 des Ringleitungssystems 6 ist in FIG. 2 für eine im Haus der Anmelderin bekannte, ungeregelte Umkehrosmoseanlage 4 detaillierter (gegenüber FIG. 1 "auf dem Kopf stehend") dargestellt. "Ungeregelt" heißt in diesem Zusammenhang, dass die Pumpe der Umkehrosmoseanlage 4, die die zu reinigende Flüssigkeit durch die Membran drückt, nicht geregelt ist und daher typischerweise während des Betriebs durchgängig auf voller Drehzahl läuft. Die Gesamtheit der in FIG. 2 dargestellten Komponenten der Rücklaufsektion 24 wird nachfolgend auch als Rücklaufvorrichtung 26 bezeichnet. Darin sind die Ringleitungsrückläufe 101, 105 die stromabwärts der Verbraucher 8 gelegenen Endabschnitte der Ringleitungen aus FIG. 1.

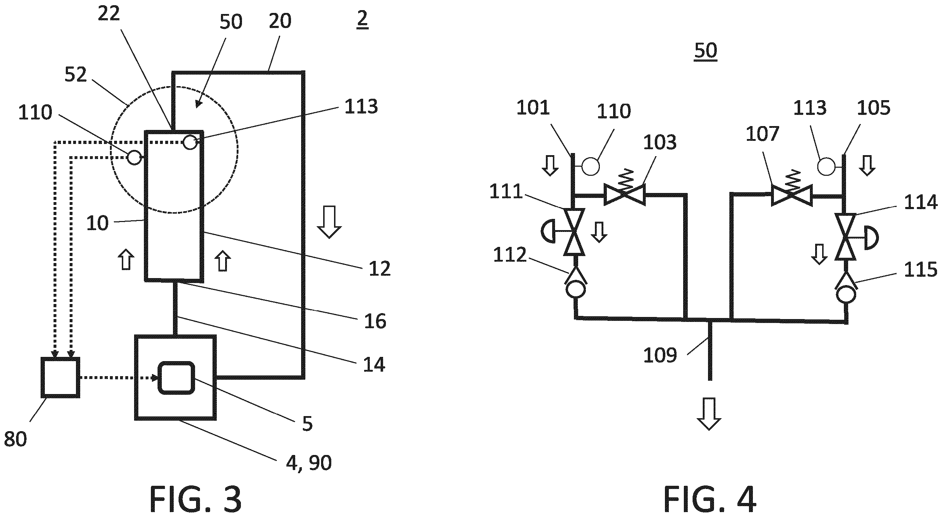

Dabei ist in den ersten Ringleitungsrücklauf 101 stromaufwärts der Vereinigung (im Permeatsammler 109, in der schematischen Übersicht gemäß FIG. 3 auch als 22 bezeichnet) mit dem zweiten Ringleitungsrücklauf 105 ein Druckhalteventil 103 eingesetzt. In analoger Ausgestaltung weist der zweite Ringleitungsrücklauf 105 ein Druckhalteventil 107 auf. Mit anderen Worten wird das Permeat im ersten Ringleitungsrücklauf 101 durch das Druckhalteventil 103 in den Permeatsammler 109 geleitet. Äquivalent wird das Permeat im zweiten Ringleitungsrücklauf 105 durch das Druckhalteventil 107 in den gleichen Permeatsammler 109 geleitet. In beiden Druckhalteventilen 103, 107 wird vorteilhafterweise der Druck des Permeats konstant gehalten, indem der Volumenstrom vom Ventil geregelt wird. Das Druckhalteventil 103, 107 weist jeweils ein integriertes Stellglied (typischerweise eine Stellschraube) auf, über das der jeweilige Sollwert des Drucks manuell einstellbar ist. Das konsolidierte Permeat wird im Anschluss durch den gemeinsamen Rücklauf 20 zurück in die Umkehrosmoseanlage 4 geleitet (siehe auch FIG. 1).

Des Weiteren weist der jeweilige Ringleitungsrücklauf 101, 105 im Sinne eines Bypasses zum Druckhalteventil 103, 107 einen strömungsmäßig parallel geschalteten Leitungsabschnitt auf, in den ein steuerbares Magnetventil 104, 108 eingesetzt ist. Während Spülprozessen werden die Magnetventile 104, 108 geöffnet, um durch den verringerten Fließwiderstand die Flussgeschwindigkeit in den Ringleitungen 10, 12 zu erhöhen. Das Öffnen wird zu Beginn des Spülvorganges durch ein elektrisches Signal von der Umkehrosmoseanlage 4 bzw. einer zugeordneten Steuerung gesteuert.

Die Nachteile der bisherigen Lösung sind unter anderem folgende:

### Einstellung der Rücklaufvorrichtung

Bevor die Umkehrosmoseanlage 4 mit der Rücklaufvorrichtung 26 in Betrieb genommen werden kann, muss diese manuell eingestellt werden. Dabei muss eine Person die Druckhalteventile 103, 107 unter Zuhilfenahme von Volumenstromsensoren 102, 106, die stromaufwärts der Druckhalteventile 103, 107 an die Ringleitungsrückläufe 101, 105 angeschlossen sind, über eine jeweilige Stellschraube einstellen. Dieser Vorgang muss getätigt werden, um die unterschiedlichen Druckverluste der verbundenen Ringleitungen 10, 12 auszugleichen. Bei einer Anpassung einer der beiden Ringleitungen 10, 12 muss dieser Vorgang jedes Mal wiederholt werden.

### Inkompatibilität mit druckgeregelten Umkehrosmoseanlagen

Durch die Funktionsweise der oben beschriebenen Druckhalteventile 103, 107 können mit der Rücklaufvorrichtung 26 keine druckgeregelten Umkehrosmoseanlagen 4 ausgestattet werden. Die Rücklaufvorrichtung 26 kann vielmehr nur für Umkehrosmoseanlagen 4 eingesetzt werden, die einen ungeregelten oder volumenstromgeregelten Betrieb zulassen.

### Kosten

Aufgrund der hohen Anforderung an thermische Beständigkeit sind die für eine Volumenstromregelung ausgelegten Druckhalteventile 103, 107 bei gewissen Varianten sehr teuer.

Zur Vermeidung der genannten Nachteile ist in FIG. 4 eine erfindungsgemäß weiterentwickelte Rücklaufvorrichtung 50 dargestellt. Die Grundkonfiguration des Gesamtsystems ist in FIG. 3 dargestellt (die Entnahmeleitungen und Verbraucher sind hier der Übersichtlichkeit halber weggelassen). Wie im Stand der Technik werden bei der Rücklaufvorrichtung 50 zwei Ringleitungen 10, 12 im jeweiligen Ringleitungsrücklauf 101, 105 über einen Permeatsammler 22 bzw. 109, hier beispielsweise in Gestalt einer Y- oder T-Vereinigung zu einem gemeinsamen Rücklauf 20 zur Umkehrosmoseanlage 4 zurückgeführt. Die Strömungsrichtung des Permeats ist durch Strömungspfeile veranschaulicht.

Die in FIG. 3 nur schematisch dargestellte Rücklaufsektion 52 des Ringleitungssystems, sprich die Rückflussvorrichtung 50 ist in FIG. 4 detaillierter dargestellt. In den Ringleitungsrücklauf 101, 105 ist jeweils ein Durchflussbegrenzer 111, 114 eingesetzt, der meist vom Hersteller für einen bestimmten Nenndurchfluss gefertigt ist und damit unveränderlich ist. Alternativ ist er mittels eines Stellglieds hinsichtlich des Durchflusses einstellbar. Damit ist druckunabhängig ein nach oben begrenzter Grunddurchsatz (Volumenstrom) von Permeat durch die jeweilige Ringleitung 10, 12 gewährleistet. Des Weiteren existiert in jedem Ringleitungsrücklauf 101, 105 nach Art einer Bypassleitung zum Durchflussbegrenzer 111, 114 ein strömungsmäßig parallel zum Durchflussbegrenzer 111, 114 angeordneter Leitungsstrang, in den ein Druckhalteventil 103, 107 eingesetzt ist. Die Druckhalteventile 103, 107 sind bevorzugt derart ausgestaltet, dass sie bei niedrigem Mediendruck geschlossen sind, bei Erreichen oder Überschreiten eines voreingestellten und zweckmäßigerweise durch ein Stellglied einstellbaren (Mindest-) Öffnungsdrucks jedoch öffnen. Dadurch wird im jeweiligen Ringleitungsrücklauf 101, 105 ein kontinuierlicher Volumenstrom bei variablem Druck erreicht und ein (stark) erhöhter Volumenstrom bei Überschreiten des Druckgrenzwertes (d. h. des voreingestellten Öffnungsdrucks) ermöglicht.

Im Gegensatz zu den Druckhalteventilen 103, 105 in der Rücklaufvorrichtung 26 gemäß FIG. 2 wird bei den Druckhalteventilen 103, 105 in der Rücklaufvorrichtung 50 gemäß FIG. 4 vorteilhafterweise der Volumenstrom nicht aktiv geregelt bzw. justiert, sondern es wird lediglich der Öffnungsdruck eingestellt. Dadurch können günstigere Ventile verwendet werden. Überdies kann bei derartigen Ventilen kein Volumenstrom entgegen der Flussrichtung erfolgen.

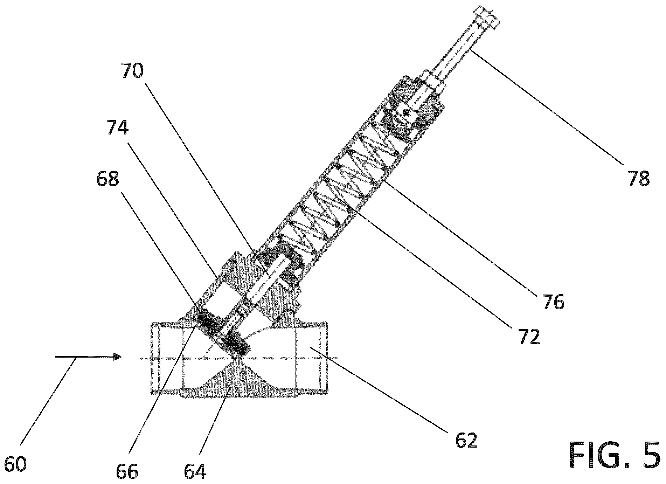

Ein Beispiel für ein in der Rücklaufvorrichtung 50 gemäß FIG. 4 einsetzbares Druckhalteventil 103, 107 (auch als Überströmventil bezeichnet) ist in FIG. 5 im Schnitt dargestellt. Ein im Beispiel in Hauptströmungsrichtung 60 von links (Primärseite) nach rechts (Sekundärseite) durchströmter Strömungskanal 62 ist in einem Schrägsitzgehäuse 64 angeordnet, welches ferner einen schräg zur Hauptströmungsrichtung 60 im Strömungskanal 62 ausgebildeten Ventilsitz 66 umfasst. Ein im geschlossenen Zustand am Ventilsitz 66 anliegender Ventilteller 68 ist über eine Kolbenstange oder Spindel 70, die eine abgedichtete Öffnung im Schrägsitzgehäuse 64 durchgreift, mit einer Druckfeder 72 verbunden, die in Verlängerung des schrägen Ventilflansches 74 in einem umgebenden Zylindergehäuse 76 angeordnet ist. Die Druckfeder 72 drückt im Schließzustand den Ventilteller 68 gegen den primärseitig anliegenden Mediumdruck in den Ventilsitz 66. Der Ventilteller 68 hebt aber vom Ventilsitz 66 ab und gibt einen Überströmweg von der Primärseite zur Sekundärseite frei, wenn die durch das Medium bewirkte Öffnungskraft die Schließkraft der Druckfeder 72 übersteigt. Über ein Stellglied 78 in Gestalt einer Stellschraube ist die Vorspannung der Druckfeder 72 und damit der Öffnungsdruck (auch Ansprechdruck genannt) des Druckhalteventils 103, 107 einstellbar.

Generell wirkt bei einem proportional arbeitenden Überströmventil dem Mediumdruck eine Feder entgegen, um das Ventil geschlossen zu halten. Derartige Ventile funktionieren ohne Fremdenergie. Wird der Ansprechdruck erreicht, beginnt das Ventil vorzugsweise proportional zur Druckerhöhung zu öffnen, bis der notwendige Hub erreicht ist und der Systemdruck sinken kann. Sofort beginnt das Ventil wieder proportional zur Druckminderung zu schließen. Knapp unterhalb des Ansprechdrucks ist das Ventil wieder komplett geschlossen. Einsetzender Rückfluss treibt das Ventil ebenfalls automatisch in den Schließzustand.

Gemäß FIG. 4 kann stromabwärts des Durchflussbegrenzers 111, 114 und stromaufwärts der Einmündung des das Druckhalteventil 103, 107 enthaltenden Leitungsstrangs ein (optionales) Rückschlagventil 112, 115 zur Verhinderung eines Rückflusses von Permeat in dem entsprechenden Strang des Ringleitungsrücklaufs 101, 105 geschaltet sein. Alternativ kann das Rückschlagventil 112, 115 stromaufwärts des Durchflussbegrenzers 111, 114 und stromabwärts der Leitungsverzweigung zum Druckhalteventil 103, 107 angeordnet sein (siehe auch weiter unten). Dadurch wird auch bei ungünstigen Einstellungen und/oder Druckverhältnissen eine Rückzirkulation von Permeat in der den Durchflussbegrenzer 111, 114 und das Druckhalteventil 103, 107 enthaltenden Leitungsschleife sicher verhindert. Die Rückschlagventile 112, 115 sind jedoch nicht zwingend erforderlich und können gegebenenfalls weggelassen werden.

Für die nachfolgend zu beschreibende Regelung der Umkehrosmoseanlage 4 ist ferner in jedem Ringleitungsrücklauf 101, 105 ein Drucksensor 110, 113 vorgesehen, der den Mediumdruck stromaufwärts des Durchflussbegrenzers 111, 114 (und des Druckhalteventils 103, 107) und vorzugsweise stromabwärts der an die Ringleitung 10, 12 angeschlossenen Verbraucher 8 erfasst. Beispielsweise kann der jeweilige Drucksensor 110, 113 kurz vor oder nach der Verzweigung des Ringleitungsrücklaufs 101, 105 in die parallelen Stränge mit dem Druckhalteventil 103, 107 einerseits und dem Durchflussbegrenzer 111, 114 andererseits an die jeweilige Rohrleitung angeschlossen oder in sie integriert sein.

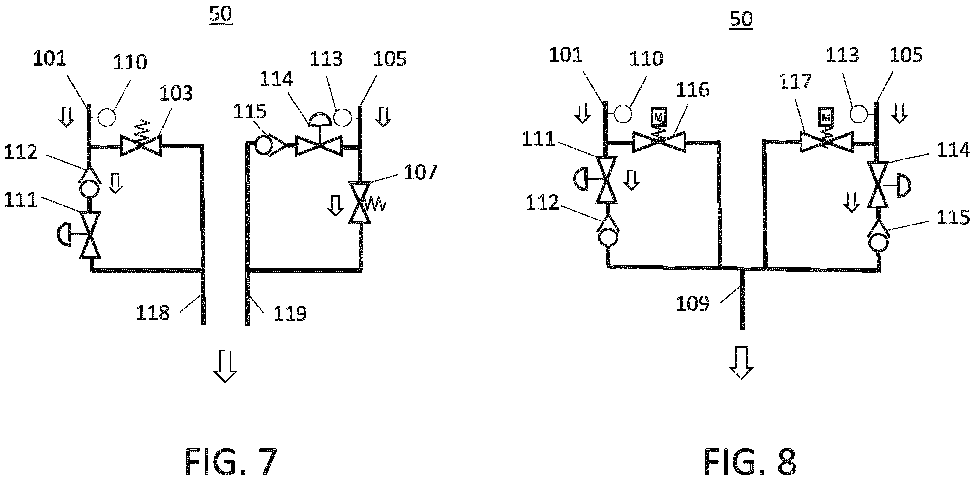

Die beiden Drucksensoren 110, 113 liefern Druckmesswerte, die als Eingangsgröße für eine druckgeregelte Umkehrosmoseanlage 4 verwendet werden. Die Anlage verwendet im Normal- oder Regelbetrieb, typischerweise während einer Dialyse, einen der beiden Druckmesswerte als Regelgröße für die in ihr enthaltene(n) Pumpe(n) 5. Die Entscheidung, welcher der beiden Druckmesswerte bzw. welcher der beiden Drucksensoren 110, 113 verwendet wird, wird in einer dem Regelbetrieb zeitlich vorangehenden Initialisierungs- oder Einstellungsphase getroffen. Das entsprechende Initialisierungsverfahren ist in FIG. 8 nach Art eines Flussdiagrams dargestellt und wird nachfolgend beschrieben:

### Schritt S1 - Starten der Pumpen im Regelbetrieb

Die Pumpe(n) 5 der Umkehrosmoseanlage 4 wird/werden im Regelbetrieb gestartet. Dabei wird der gemessene Druck einer der Drucksensoren 110, 113 als Prozessvariable für die initiale Pumpenregelung angewendet. Es ist dabei egal, welcher der beiden Drucksensoren 110, 113 initial verwendet wird. Es ist daher beispielsweise der Einfachheit halber möglich, immer denselben Drucksensor 110 oder 113 auszuwählen.

### Schritt S2 - Messen des Drucks mit den Drucksensoren 110, 113

Der von den Drucksensoren 110, 113 gemessene Druck wird über einen Auswertungszeitraum / Messzeitraum gegeneinander verglichen. Der Vergleich findet entweder über einen gemittelten Druckmesswert über den gesamten Auswertungszeitraum oder iterativ während des Auswertungszeitraumes statt.

### Entscheidung D1 - Auswahl des Drucksensors 100, 113

Der Drucksensor 110, 113, welcher den niedrigeren Druck während des Auswertungs- bzw. Beobachtungszeitraumes ermittelt hat, wird als Prozessvariable ausgewählt. Exemplarisch wird hierfür in diesem Schritt die Frage gestellt, ob der vom Drucksensor 110 ermittelte Druckmesswert niedriger ist als der vom Drucksensor 113 ermittelte Druckmesswert.

Je nach Ausgang der Entscheidung bzw. Ermittlung folgt nun Schritt S3 oder S4:

### Schritt S3 - Verwendung des Drucksensors 110

Der Drucksensor 110 mit dem niedrigeren Druck in der Initialisierungsphase bzw. dessen Druckmesswert wird als Prozessvariable für die Pumpenregelung der Umkehrosmoseanlage 4 verwendet.

### Schritt S4 - Verwendung des Drucksensors 113

Der Drucksensor 113 mit dem niedrigeren Druck in der Initialisierungsphase bzw. dessen Druckmesswert wird als Prozessvariable für die Pumpenregelung der Umkehrosmoseanlage 4 verwendet.

Der ausgewählte Drucksensor 110, 113 wird bis zu einem vorab festgelegten Ende der Pumpensteuerung verwendet, typischerweise bis zur Beendigung eines aktuellen Dialysevorgangs. Nach einer Spülung der Anlage startet der Vorgang von neuem.

Wie bereits erwähnt, wird das Initialisierungsverfahren zur Identifikation des entsprechenden Drucksensors 110, 113 vor der eigentlichen Dialyse durchgeführt. Das bedeutet, dass während der Identifikation keine äußeren Störeinflüsse auf das System einwirken sollen. Nachdem die Identifikation abgeschlossen ist, kann die druckgeregelte Umkehrosmoseanlage 4 zwei Ringleitungen 10, 12 bedarfsgerecht mit Permeat versorgen. Durch die Auswahl des Drucksensors 110, 113 mit dem niedrigeren Druckmesswert als Prozessvariable für die Pumpensteuerung der Umkehrosmoseanlage 4 wird im Sinne der Versorgungssicherheit ein zum reibungslosen Betrieb erforderlicher Mindestdurchsatz von Permeat durch die beiden Ringleitungen 10, 12 sichergestellt.

Je nachdem, wie unterschiedlich die Ringleitungen 10, 12 in Bezug auf den Druckverlust sind, kann die Energieeffizienz der druckgeregelten Umkehrosmoseanlage 4 sinken, da ein kontinuierlicher Volumenstrom über das jeweilige Druckhalteventil 103, 107 (Überströmventil) fließt. Dies kann entweder durch manuelles Nachjustieren der Schließkraft des jeweiligen Druckhalteventils 103, 107 nachgebessert oder vollständig ignoriert werden.

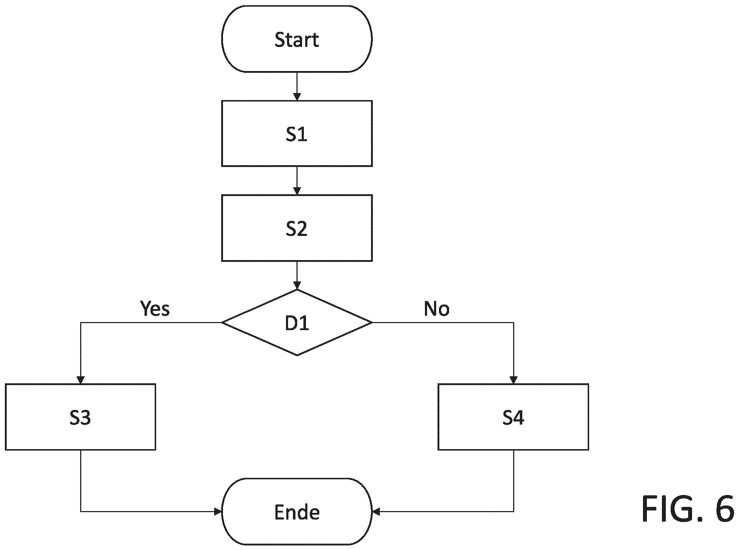

Die beschriebene Vorrichtung und das zugehörige Verfahren lassen verschiedene Ausgestaltungen, Abwandlungen und Verallgemeinerungen zu, die nachfolgend exemplarisch beschreiben werden:
Das mit Bezug auf FIG. 6 beschriebene Initialisierungsverfahren und/oder der nachfolgende Regelbetrieb können mittels Hardware und/oder Software durch eine Steuer- oder Regeleinheit 80 durchgeführt werden, die beispielsweise in die Umkehrosmoseanlage 4 integriert ist oder eine diesbezüglich externe Komponente bildet (siehe auch FIG. 3, worin die Wirkzusammenhänge durch gestrichelte Pfeile angedeutet sind).

Wie bereits erwähnt, können die Vorrichtung und das Verfahren für mehr als zwei Ringleitungen 10, 12 ausgelegt sein. Der Permeatteiler 16 und die Rücklaufvorrichtung 50 können dementsprechend beliebig erweitert werden. Das beschriebene Prinzip der Initialisierung und Steuerung / Regelung, wonach derjenige Drucksensor 110, 113 als Prozessvariable für die Pumpensteuerung ausgewählt wird, in dessen Ringleitung 10, 12 der geringste Druck (und damit der höchste Druckverlust) herrscht, bleibt erhalten.

Die Rückschlagventile 112, 115 sind nicht zwingend erforderlich und können bei Bedarf weggelassen werden. Falls vorhanden, kann das jeweilige Rückschlagventil 112, 115 statt stromabwärts des Durchflussbegrenzers 111, 114 stromaufwärts desselben (aber weiterhin in Reihe zu diesem) in dem zum Druckhalteventil 103, 107 parallelen Leitungsstrang angeordnet sein.

Des Weiteren können die wenigstens zwei Ringleitungen 10, 12 im jeweiligen Ringleitungsrücklauf 101, 105 auch separat in einen Tank geführt werden und müssen nicht zwangsläufig (wie in FIG. 4) über einen Permeatsammler 109 zusammengeführt werden. Eine derartige Variante der Rücklaufvorrichtung 50 mit jeweils separater Tankrückführung des Permeats über einen ersten und zweiten Permeatrücklauf 118, 119 (und hier auch mit unterschiedlich angeordneten Rückschlagventilen 112, 115) ist in FIG. 7 dargestellt.

Generell können die Komponenten im Ringleitungsrücklauf variiert werden, solange folgende Anforderungen erfüllt sind:
- Kontinuierlicher Volumenstrom bei variablem Druck
- (Stark) erhöhter Volumenstrom bei Überschreiten eines Druckgrenzwertes

Als weitere Ausführungsvariante können in der Rücklaufvorrichtung 50 anstelle von regulären, manuell einstellbaren Druckhalteventilen 103, 107 elektrisch justierbare Druckhalteventile oder Überströmventile 116, 117 zum Einsatz kommen. Eine derartige Variante ist in FIG. 8 dargestellt. Die dort dargestellte Rücklaufvorrichtung 50 weist solche elektrisch justierbaren Überströmventile 116, 117 mit jeweiligem Einstellantrieb auf, entspricht im Übrigen aber der Grundversion aus FIG. 4.

Die Pumpensteuerung für die Umkehrosmoseanlage 4 kann damit - insbesondere bei konstant bleibender Einstellung der Überströmventile 116, 117 - grundsätzlich genauso ablaufen wie bislang beschrieben, nämlich durch Auswahl des Drucksensors 110, 113 mit dem niedrigsten gemessenen Druck während einer Initialisierungsphase. Unter Ausnutzung der Ansteuerbarkeit und elektrischen Verstellbarkeit der Überströmventile 116, 117 hinsichtlich ihres Halte- bzw. Öffnungsdrucks ist jedoch eine Abwandlung des Verfahrens vorteilhaft, bei der der Auswahlprozess des Drucksensors 110, 113 entfällt und stattdessen die Überströmventile 116, 117 so eingestellt werden, dass die beiden Drücke in allen Ringleitungsrückläufen 101, 105 identisch sind. Das bedeutet, dass der Haltedruck des jeweiligen Überströmventils 116, 117 so lange reduziert wird, bis der gewollte Druck am Ringleitungsende vorherrscht. Dabei können die Einstellbereiche der Überströmventile 116, 117 so limitiert werden, dass die Anlage immer noch problemlos funktioniert. Das bedeutet, ein vorgegebener minimaler Druck und/oder Volumenstrom darf nicht unterschritten, und ein vorgegebener maximaler Druck und/oder Volumenstrom darf nicht überschritten werden.

Sollte ein identisches Einstellen des Drucks innerhalb des vorgegebenen Bereiches nicht möglich sein, muss stattdessen wieder das oben beschriebene Drucksensor-Auswahlverfahren durchgeführt werden.

In Verallgemeinerung der bisherigen Beschreibung kann anstelle einer Umkehrosmoseanlage 4 auch eine andere Wasseraufbereitungsanlage 90 zur Erzeugung von Permeat zum Einsatz kommen.

### Bezugszeichenliste

- 2: Permeatversorgungssystem
- 4: Umkehrosmoseanlage
- 5: Pumpe
- 6: Ringleitungssystem
- 8: Verbraucher
- 10: Ringleitung
- 12: Ringleitung
- 14: Vorlauf
- 16: Permeatteiler
- 18: Entnahmeleitung
- 20: Rücklauf
- 22: Permeatsammler
- 24: Rücklaufsektion
- 26: Rücklaufvorrichtung
- 50: Rücklaufvorrichtung
- 52: Rücklaufsektion
- 60: Hauptströmungsrichtung
- 62: Strömungskanal
- 64: Schrägsitzgehäuse
- 66: Ventilsitz
- 68: Ventilteller
- 70: Spindel
- 72: Druckfeder
- 74: Ventilflansch
- 76: Zylindergehäuse
- 78: Stellglied
- 80: Steuer- oder Regeleinheit
- 90: Wasseraufbereitungsanlage
- 101: Ringleitungsrücklauf
- 102: Volumenstromsensor
- 103: Druckhalteventil
- 104: Magnetventil (Spülung)
- 105: Ringleitungsrücklauf
- 106: Volumenstromsensor
- 107: Druckhalteventil
- 108: Magnetventil (Spülung)
- 109: Permeatsammler
- 110: Drucksensor
- 111: Durchflussbegrenzer
- 112: Rückschlagventil
- 113: Drucksensor
- 114: Durchflussbegrenzer
- 115: Rückschlagventil
- 116: Überströmventil (elektrisch justierbar)
- 117: Überströmventil (elektrisch justierbar)
- 118: Permeatrücklauf der ersten Ringleitung
- 119: Permeatrücklauf der zweiten Ringleitung

## Patentansprüche

1. Permeatversorgungssystem (2) für medizinische Anwendungen, umfassend
• eine Wasseraufbereitungsanlage (90) mit mindestens einer integrierten Pumpe (5) zur Produktion von Permeat,
• ein eingangsseitig über einen Permeatteiler (16) an die Wasseraufbereitungsanlage (90) angeschlossenes Ringleitungssystem mit mindestens zwei parallel geschalteten Ringleitungen (10, 12), die ausgangsseitig über eine Rücklaufvorrichtung (50) an die Wasseraufbereitungsanlage (90) angeschlossen sind,
• eine Steuer- oder Regeleinheit (80),
wobei die Rücklaufvorrichtung (50) für jede der Ringleitungen (10, 12) folgende Komponenten umfasst:
• einen Ringleitungsrücklauf (101, 105), der sich in zwei parallel geschaltete Leitungsstränge verzweigt, wobei der eine Leitungsstrang einen Durchflussbegrenzer (111, 114) und der andere Leitungsstrang ein oberhalb eines eingestellten Öffnungsdrucks öffnendes Druckhalteventil (103, 107) aufweist,
• einen Drucksensor (110, 113), der stromaufwärts des Durchflussbegrenzers (111, 114) und des Druckhalteventils (103, 107) den Druck im Ringleitungsrücklauf (101, 105) misst,
und wobei in der Steuer- oder Regeleinheit (80) ein Steuer- oder Regelalgorithmus implementiert ist, der im Normalbetrieb anhand eines von einem der Drucksensoren (110, 113) gemessenen Drucks die Pumpe (5) ansteuert.

2. Permeatversorgungssystem (2) nach Anspruch 1, wobei der Steuer- oder Regelalgorithmus so beschaffen ist, dass er in einer Initialisierungsphase genau einen der Drucksensoren (110, 113) für die Steuerung der Pumpe (5) im anschließenden Normalbetrieb auswählt.

3. Permeatversorgungssystem (2) nach Anspruch 2, wobei der ausgewählte Drucksensor (110, 113) derjenige ist, der in der Initialisierungsphase den niedrigsten Druck misst.

4. Permeatversorgungssystem (2) nach einem der vorangehenden Ansprüche, wobei die Ringleitungsrückläufe (101, 105) über einen Permeatsammler (109) in einem gemeinsamen Rücklauf (20) münden.

5. Permeatversorgungssystem (2) nach einem der vorangehenden Ansprüche, wobei in den Leitungsstrang mit dem Durchflussbegrenzer (111, 114) ein Rückschlagventil (112, 115) geschaltet ist.

6. Permeatversorgungssystem (2) nach einem der vorangehenden Ansprüche, wobei das Druckhalteventil (103, 107) ein Überströmventil ist und eine auf einen Ventilteller (68) einwirkende Feder, insbesondere eine Druckfeder (72) aufweist, die dem Mediumdruck entgegenwirkt, um unterhalb des Öffnungsdrucks das Überströmventil geschlossen zu halten.

7. Permeatversorgungssystem (2) nach Anspruch 1, wobei die Druckhalteventile (103, 107) hinsichtlich ihres Öffnungsdrucks elektrisch justierbar sind und der Steuer- oder Regelalgorithmus derart auf die Druckhalteventile (103, 107) einwirkt, dass der gleiche Druck in allen Ringleitungsrückläufen (101, 105) angestrebt wird.

8. Permeatversorgungssystem (2) nach einem der vorangehenden Ansprüche, wobei die Wasseraufbereitungsanlage (90) eine Umkehrosmoseanlage (4) ist.

9. Verfahren zum Betreiben eines Permeatversorgungssystems (2) für medizinische Anwendungen, umfassend
• eine Wasseraufbereitungsanlage (90) mit mindestens einer integrierten Pumpe (5) zur Produktion von Permeat,
• ein eingangsseitig über einen Permeatteiler (16) an die Wasseraufbereitungsanlage (90) angeschlossenes Ringleitungssystem mit mindestens zwei parallel geschalteten Ringleitungen (10, 12), die ausgangsseitig über eine Rücklaufvorrichtung (50) an die Wasseraufbereitungsanlage (90) angeschlossen sind,
wobei die Rücklaufvorrichtung (50) für jede der Ringleitungen (10, 12) folgende Komponenten umfasst:
• einen Ringleitungsrücklauf (101, 105), der sich in zwei parallel geschaltete Leitungsstränge verzweigt, wobei der eine Leitungsstrang einen Durchflussbegrenzer (111, 114) und der andere Leitungsstrang ein oberhalb eines eingestellten Öffnungsdrucks öffnendes Druckhalteventil (103, 107) aufweist,
• einen Drucksensor (110, 113), der stromaufwärts des Durchflussbegrenzers (111, 114) und des Druckhalteventils (103, 107) den Druck im Ringleitungsrücklauf (101, 105) misst,
und wobei im Normalbetrieb ein von einem der Drucksensoren (110, 113) gemessener Druck als Prozessvariable für die Ansteuerung der Pumpe (5) verwendet wird.

10. Verfahren nach Anspruch 9, wobei in einer Initialisierungsphase genau einer der Drucksensoren (110, 113) für die Steuerung der Pumpe (5) im anschließenden Normalbetrieb ausgewählt wird.

11. Verfahren nach Anspruch 10, wobei der derjenige Drucksensor (110, 113) ausgewählt wird, der in der Initialisierungsphase den niedrigsten Druck misst.

12. Verfahren nach Anspruch 9 mit folgenden Schritten:
• Starten der Pumpe (5) der Wasseraufbereitungsanlage (90), wobei einer der Drucksensoren (110, 113) für eine initiale Pumpensteuerung verwendet wird,
• Messen des Drucks mit den Drucksensoren (110, 113) über einen Auswertungszeitraum hinweg und Vergleichen der so erhaltenen Druckmesswerte der beiden Drucksensoren (110,113),
• Auswählen des Drucksensors (110, 113), welcher den niedrigsten Druck während des Auswertungszeitraumes ermittelt hat,
• Verwenden des ausgewählten Drucksensors (110, 113) als Prozessvariable für die Pumpensteuerung.
